# EUROPEAN PATENT SPECIFICATION

(11) **EP 1 035 860 B1**
(45) Date of publication and mention of the grant of the patent: **10.05.2006**
(21) Application number: 98960439.2
(22) Date of filing: 01.12.1998
(51) Int. Cl.: A61K 38/00, C07H 21/04, C07K 1/00, C07K 2/00

(54) **NON-TOXIC IMMUNE STIMULATING ENTEROTOXIN COMPOSITIONS**
NICHT-TOXISCHE IMMUN-STIMULIERENDE ENTEROTOXIN-ZUSAMMENSETZUNGEN
COMPOSITIONS D'ENTEROTOXINES NON TOXIQUES STIMULANT LE SYSTEME IMMUNITAIRE

(30) Priority: 02.12.1997 US 67357 P
(43) Date of publication of application: 20.09.2000
(73) Proprietor: IDAHO RESEARCH FOUNDATION, INC., Moscow, Idaho 83844-3003 (US)
(72) Inventor: BOHACH, Gregory, I., Moscow, ID 83844 (US)
(74) Representative: Leifert, Elmar
(86) International application number: PCT/US1998/025107
(87) International publication number: WO 1999/027889

(56) References cited:
- WO-A1-97/36932
- HOVDE C J ET AL: "INVESTIGATION OF THE ROLE OF THE DISULPHIDE BOND IN THE ACTIVITY AND STRUCTURE OF STAPHYLOCOCCAL ENTEROTOXIN C1" MOLECULAR MICROBIOLOGY,GB,BLACKWELL SCIENTIFIC, OXFORD, vol. 13, no. 5, 1994, pages 897-909, XP002067000 ISSN: 0950-382X
- NISHI et al., "B Cell Epitope Mapping of the Bacterial Superantigen Staphylococcal Enterotoxin B", JOURNAL OF IMMUNOLOGY, 01 January 1997, Vol. 158, No. 1, pages 247-254, XP002918943
- ALAKHOV et al., "Identification of Functionally Active Fragments of Staphylococcal Enterotoxin B", EUR. J. BIOCHEM., 01 November 1992, Vol. 209, pages 823-828, XP002918944
- BINEK et al., "Localisation of the Mitogenic Epitope of Staphylococcal Enterotoxin B", J. MED. MICROBIOL., March 1992, Vol. 36, pages 156-163, XP002918945

### SEQUENCE LISTING

<110> IDAHO RESEARCH FOUNDATION, INC.
<120> NON-TOXIC IMMUNE STIMULATING ENTEROTOXIN COMPOSITIONS
<130> 12136. 1EPWO
<140> 98960439.2
   <141> 1998-12-01
<150> PCT/US98/25107
   <151> 1998-12-01
<150> 60/067,357
   <151> 1997-12-02
<160> 66
<170> PatentIn Ver. 2.1
<210> 1
   <211> 240
   <212> PRT
   <213> Staphylococcus aureus
<220>
   <221> UNSURE
   <222> (240)
   <223> Xaa is unknown.
<400> 1
   Glu Ser Gln Pro Asp Pro Thr Pro Asp Glu Leu His Lys Ala Ser Lys
     1               5                  10                  15
   Phe Thr Gly Leu Met Glu Asn Met Lys Val Leu Tyr Asp Asp His Tyr
                20                  25                  30
   Val Ser Ala Thr Lys Val Lys Ser Val Asp Lys Phe Leu Ala His Asp
            35                  40                  45
   Leu Ile Tyr Asn Ile Ser Asp Lys Lys Leu Lys Asn Tyr Asp Lys Val
        50                  55                  60
   Lys Thr Glu Leu Leu Asn Glu Gly Leu Ala Lys Lys Tyr Lys Asp Glu
    65                  70                  75                  80
   Val Val Asp Val Tyr Gly Ser Asn Tyr Tyr Val Asn Cys Tyr Phe Ser
                    85                  90                  95
   Ser Lys Asp Asn Val Gly Lys Val Thr Gly Gly Lys Thr Cys Met Tyr
               100                 105                 110
   Gly Gly Ile Thr Lys His Glu Gly Asn His Phe Asp Asn Gly Asn Leu
           115                 120                 125
   Gln Asn Val Leu Ile Arg Val Tyr Glu Asn Lys Arg Asn Thr Ile Ser
       130                 135                 140
   Phe Glu Val Gln Thr Asp Lys Lys Ser Val Thr Ala Gln Glu Leu Asp
   145                 150                 155                 160
   Ile Lys Ala Arg Asn Phe Leu Ile Asn Lys Lys Asn Leu Tyr Glu Phe
                   165                 170                 175
   Asn Ser Ser Pro Tyr Glu Thr Gly Tyr Ile Lys Phe Ile Glu Asn Asn
               180                 185                 190
   Gly Asn Thr Phe Trp Tyr Asp Met Met Pro Ala Pro Gly Asp Lys Phe
           195                 200                 205
   Asp Gln Ser Lys Tyr Leu Met Met Tyr Asn Asp Asn Lys Thr Val Asp
       210                 215                 220
   Ser Lys Ser Val Lys Ile Glu Val His Leu Thr Thr Lys Asn Gly Xaa
   225                 230                 235                 240
<210> 2
   <211> 240
   <212> PRT
   <213> Staphylococcus aureus
<220>
   <221> UNSURE
   <222> (240)
   <223> Xaa is unknown.
<400> 2
   Glu Ser Gln Pro Asp Pro Thr Pro Asp Glu Leu His Lys Ser Ser Glu
   1               5                   10                  15
   Phe Thr Gly Thr Met Gly Asn Met Lys Tyr Leu Tyr Asp Asp His Tyr
               20                  25                  30
   Val Ser Ala Thr Lys Val Met Ser Val Asp Lys Phe Leu Ala His Asp
           35                  40                  45
   Leu Ile Tyr Asn Ile Ser Asp Lys Lys Leu Lys Asn Tyr Asp Lys Val
       50                  55                  60
   Lys Thr Glu Leu Leu Asn Glu Asp Leu Ala Lys Lys Tyr Lys Asp Glu
   65                  70                  75                  80
   Val Val Asp Val Tyr Gly Ser Asn Tyr Tyr Val Asn Cys Tyr Phe Ser
                   85                  90                  95
   Ser Lys Asp Asn Val Gly Lys Val Thr Gly Gly Lys Thr Cys Met Tyr
               100                 105                 110
   Gly Gly Ile Thr Lys His Glu Gly Asn His Phe Asp Asn Gly Asn Leu
           115                 120                 125
   Gln Asn Val Leu Ile Arg Val Tyr Glu Asn Lys Arg Asn Thr Ile Ser
       130                 135                 140
   Phe Glu Val Gln Thr Asp Lys Lys Ser Val Thr Ala Gln Glu Leu Asp
   145                 150                 155                 160
   Ile Lys Ala Arg Asn Phe Leu Ile Asn Lys Lys Asn Leu Tyr Glu Phe
                   165                 170                 175
   Asn Ser Ser Pro Tyr Glu Thr Gly Tyr Ile Lys Phe Ile Glu Asn Asn
               180                 185                 190
   Gly Asn Thr Phe Gln Tyr Asp Met Met Pro Ala Pro Gly Asp Lys Phe
           195                 200                 205
   Asp Gln Ser Lys Tyr Leu Met Met Tyr Asn Asp Asn Lys Thr Val Asp
       210                 215                 220
   Ser Lys Ser Val Lys Ile Glu Val His Leu Thr Thr Lys Asn Gly Xaa
   225                 230                 235                 240
<210> 3
   <211> 240
   <212> PRT
   <213> Staphylococcus aureus
<220>
   <221> UNSURE
   <222> (240)
   <223> Xaa is unknown.
<400> 3
   Glu Ser Gln Pro Asp Pro Met Pro Asp Asp Leu His Lys Ser Ser Glu
    1               5                  10                  15
   Phe Thr Gly Thr Met Gly Asn Met Lys Tyr Leu Tyr Asp Asp His Tyr
               20                  25                  30
   Val Ser Ala Thr Lys Val Lys Ser Val Asp Lys Phe Leu Ala His Asp
           35                  40                  45
   Leu Ile Tyr Asn Ile Ser Asp Lys Lys Leu Lys Asn Tyr Asp Lys Val
       50                  55                  60
   Lys Thr Glu Leu Leu Asn Glu Asp Leu Ala Lys Lys Tyr Lys Asp Glu
   65                  70                  75                  80
   Val Val Asp Val Tyr Gly Ser Asn Tyr Tyr Val Asn Cys Tyr Phe Ser
                   85                  90                  95
   Ser Lys Asp Asn Val Gly Lys Val Thr Gly Gly Lys Thr Cys Met Tyr
                   100                      105                      110
   Gly Gly Ile Thr Lys His Glu Gly Asn His Phe Asp Asn Gly Asn Leu
               115                     120                     125
   Gln Asn Val Leu Val Arg Val Tyr Glu Asn Lys Arg Asn Thr Ile Ser
       130                     135                     140
   Phe Glu Val Gln Thr Asp Lys Lys Ser Val Thr Ala Gln Glu Leu Asp
   145                     150                     155                     160
   Ile Lys Ala Arg Asn Phe Leu Ile Asn Lys Lys Asn Leu Tyr Glu Phe
                   165                     170                     175
   Asn Ser Ser Pro Tyr Glu Thr Gly Tyr Ile Lys Phe Ile Glu Asn Asn
                   180                     185                     190
   Gly Asn Thr Phe Gln Tyr Asp Met Met Pro Ala Pro Gly Asp Lys Phe
               195                     200                     205
   Asp Gln Ser Lys Tyr Leu Met Met Tyr Asn Asp Asn Lys Thr Val Asp
       210                     215                     220
   Ser Lys Ser Val Lys Ile Glu Val His Leu Thr Thr Lys Asn Gly Xaa
   225                     230                     235                     240
<210> 4
   <211> 240
   <212> PRT
   <213> Staphylococcus aureus
<220>
   <221> UNSURE
   <222> (240)
   <223> Xaa is unknown.
<400> 4
   Glu Ser Gln Pro Asp Pro Met Pro Asp Asp Leu His Lys Ser Ser Glu
   1 5 10 15
   Phe Thr Gly Thr Met Gly Asn Met Lys Tyr Leu Tyr Asp Asp His Tyr
   20 25 30
   Val Ser Ala Thr Lys Val Lys Ser Val Asp Lys Phe Leu Ala His Asp
   35 40 45
   Leu Ile Tyr Asn Ile Asn Asp Lys Lys Leu Asn Asn Tyr Asp Lys Val
   50 55 60
   Lys Thr Glu Leu Leu Asn Glu Asp Leu Ala Asn Lys Tyr Lys Asp Glu
   65 70 75 80
   Val Val Asp Val Tyr Gly Ser Asn Tyr Tyr Val Asn Cys Tyr Phe Ser
   85 90 95
   Ser Lys Asp Asn Val Gly Lys Val Thr Ser Gly Lys Thr Cys Met Tyr
   100 105 110
   Gly Gly Ile Thr Lys His Glu Gly Asn His Phe Asp Asn Gly Asn Leu
   115 120 125
   Gln Asn Val Leu Ile Arg Val Tyr Glu Asn Lys Arg Asn Thr Ile Ser
   130 135 140
   Phe Glu Val Gln Thr Asp Lys Lys Ser Val Thr Ala Gln Glu Leu Asp
   145 150 155 160
   Ile Lys Ala Arg Asn Phe Leu Ile Asn Lys Lys Asn Leu Tyr Glu Phe
   165 170 175
   Asn Ser Ser Pro Tyr Glu Thr Gly Tyr Ile Lys Phe Ile Glu Ser Asn
   180 185 190
   Gly Asn Thr Phe Trp Tyr Asp Met Met Pro Ala Pro Gly Asp Lys Phe
   195 200 205
   Asp Gln Ser Lys Tyr Leu Met Ile Tyr Lys Asp Asn Lys Met Val Asp
   210 215 220
   Ser Lys Ser Val Lys Ile Glu Val His Leu Thr Thr Lys Asn Gly Xaa
   225 230 235 240
<210> 5
   <211> 240
   <212> PRT
   <213> Staphylococcus aureus
<220>
   <221> UNSURE
   <222> (240)
   <223> Xaa is unknown.
<400> 5
   Glu Ser Gln Pro Asp Pro Thr Pro Asp Glu Leu His Lys Ser Ser Glu
    1               5                  10                  15
   Phe Thr Gly Thr Met Gly Asn Met Lys Tyr Leu Tyr Asp Asp His Tyr
               20                  25                  30
   Val Ser Ala Thr Lys Val Lys Ser Val Asp Lys Phe Leu Ala His Asp
           35                  40                  45
   Leu Ile Tyr Asn Ile Ser Asp Lys Lys Leu Lys Asn Tyr Asp Lys Val
       50                  55                  60
   Lys Thr Glu Leu Leu Asn Glu Asp Leu Ala Lys Lys Tyr Lys Asp Glu
   65                  70                  75                  80
   Val Val Asp Val Tyr Gly Ser Asn Tyr Tyr Val Asn Cys Tyr Phe Ser
                   85                  90                  95
   Ser Lys Asp Asn Val Gly Lys Val Thr Gly Gly Lys Thr Cys Met Tyr
               100                 105                 110
   Gly Gly Ile Thr Lys His Glu Gly Asn His Phe Asp Asn Gly Asn Leu
           115                 120                 125
   Gln Asn Val Leu Ile Arg Val Tyr Glu Asn Lys Arg Asn Thr Ile Ser
       130                 135                 140
   Phe Glu Val Gln Thr Asp Lys Lys Ser Val Thr Ala Gln Glu Leu Asp
   145                 150                 155                 160
   Ile Lys Ala Arg Asn Phe Leu Ile Asn Lys Lys Asn Leu Tyr Glu Phe
                   165                 170                 175
   Asn Ser Ser Pro Tyr Glu Thr Gly Tyr Ile Lys Phe Ile Glu Asn Asn
               180                 185                 190
   Gly Asn Thr Phe Gln Tyr Asp Met Met Pro Ala Pro Gly Asp Lys Phe
           195                 200                 205
   Asp Gln Ser Lys Tyr Leu Met Met Tyr Asn Asp Asn Lys Thr Val Asp
       210                 215                 220
   Ser Lys Arg Val Lys Ile Glu Val His Leu Thr Thr Lys Asn Gly Xaa
   225                 230                 235                 240
<210> 6
   <211> 240
   <212> PRT
   <213> Staphylococcus aureus
<220>
   <221> UNSURE
   <222> (240)
   <223> Xaa is unknown.
<400> 6
   Glu Ser Gln Pro Asp Pro Met Pro Asp Asp Leu His Lys Ser Ser Glu
    1               5                  10                  15
   Phe Thr Gly Thr Met Gly Asn Met Lys Tyr Leu Tyr Asp Asp His Tyr
               20                  25                  30
   Val Ser Ala Thr Lys Val Lys Ser Val Asp Lys Phe Leu Ala His Asp
           35                  40                  45
   Leu Ile Tyr Asn Ile Ser Asp Lys Arg Leu Lys Asn Tyr Asp Lys Val
           50                  55                  60
   Lys Thr Glu Leu Leu Asn Glu Asp Leu Ala Lys Lys Tyr Lys Asp Glu
    65                 70                  75                  80
   Val Val Asp Val Tyr Gly Ser Asn Tyr Tyr Val Asn Cys Tyr Phe Ser
                    85                  90                  95
   Ser Lys Asp Asn Val Gly Lys Val Thr Gly Gly Lys Thr Cys Met Tyr
               100                 105                 110
   Gly Gly Ile Thr Lys His Glu Gly Asn His Phe Asp Asn Gly Asn Leu
           115                 120                 125
   Gln Asn Val Leu Val Arg Val Tyr Glu Asn Lys Arg Asn Thr Ile Ser
       130                 135                 140
   Phe Glu Val Gln Thr Asp Lys Lys Ser Val Thr Ala Gln Glu Leu Asp
   145                 150                 155                 160
   Ile Lys Ala Arg Asn Phe Leu Ile Asn Lys Lys Asn Leu Tyr Glu Phe
                   165                 170                 175
   Asn Ser Ser Pro Tyr Glu Thr Gly Tyr Ile Lys Phe Ile Glu Asn Asn
               180                 185                 190
   Gly Asn Thr Phe Gln Tyr Asp Met Met Pro Ala Pro Gly Asp Lys Phe
           195                 200                 205
   Asp Gln Ser Lys Tyr Leu Met Met Tyr Asn Asp Asn Lys Thr Val Asp
       210                 215                 220
   Ser Lys Arg Val Lys Ile Glu Val His Leu Thr Thr Lys Asn Gly Xaa
   225                 230                 235                 240
<210> 7
   <211> 240
   <212> PRT
   <213> Staphylococcus aureus
<220>
   <221> UNSURE
   <222> (240)
   <223> Xaa is unknown.
<400> 7
   Glu Ser Gln Pro Asp Pro Thr Pro Asp Glu Leu His Lys Ala Ser Lys
   1               5                   10                  15
   Phe Thr Gly Leu Met Glu Asn Met Lys Val Leu Tyr Asp Asp Arg Tyr
               20                  25                  30
   Val Ser Ala Thr Lys Val Lys Ser Val Asp Lys Phe Leu Ala His Asp
   35 40 45
   Leu Ile Tyr Asn Ile Ser Asp Lys Lys Leu Lys Asn Tyr Asp Lys Val
   50 55 60
   Lys Thr Glu Leu Leu Asn Glu Asp Leu Ala Lys Lys Tyr Lys Asp Glu
   65 70 75 80
   Val Val Asp Val Tyr Gly Ser Asn Tyr Tyr Val Asn Cys Tyr Phe Phe
   85 90 95
   Ser Lys Asp Asn Val Gly Lys Val Thr Gly Gly Lys Thr Cys Met Tyr
   100 105 110
   Gly Gly Ile Thr Lys His Glu Gly Asn His Phe Asp Asn Gly Asn Leu
   115 120 125
   Gln Asn Val Leu Ile Arg Val Tyr Glu Asn Lys Arg Asn Thr Ile Ser
   130 135 140
   Phe Glu Val Gln Thr Asp Lys Lys Ser Val Thr Ala Gln Glu Leu Asp
   145 150 155 160
   Ile Lys Ala Arg Asn Phe Leu Ile Asn Lys Lys Asn Leu Tyr Glu Phe
   165 170 175
   Asn Ser Ser Pro Tyr Glu Thr Gly Tyr Ile Lys Phe Ile Glu Asn Asn
   180 185 190
   Gly Asn Thr Phe Gln Tyr Asp Met Met Pro Ala Pro Gly Asp Lys Phe
   195 200 205
   Asp Gln Ser Lys Tyr Leu Met Met Tyr Asn Asp Asn Lys Thr Val Asp
   210 215 220
   Ser Lys Arg Val Lys Ile Glu Val His Leu Thr Thr Lys Asn Gly Xaa
   225 230 235 240
<210> 8
   <211> 240
   <212> PRT
   <213> Staphylococcus aureus
<220>
   <221> UNSURE
   <222> (240)
   <223> Xaa is unknown.
<400> 8
   Glu Ser Gln Pro Asp Pro Thr Pro Asp Glu Leu His Lys Ala Ser Lys
    1               5                  10                  15
   Phe Thr Gly Leu Met Glu Asn Met Lys Val Leu Tyr Asp Asp Arg Tyr
               20                  25                  30
   Val Ser Ala Thr Lys Val Lys Ser Val Asp Lys Phe Leu Ala His Asp
           35                  40                  45
   Leu Ile Tyr Asn Ile Ser Asp Lys Lys Leu Lys Asn Tyr Asp Lys Val
       50                  55                  60
   Lys Thr Glu Leu Leu Asn Glu Asp Leu Ala Lys Lys Tyr Lys Asp Glu
   65                  70                  75                  80
   Val Val Asp Val Tyr Gly Ser Asn Tyr Tyr Val Asn Cys Cys Phe Phe
                   85                  90                  95
   Ser Lys Asp Asn Val Gly Lys Val Thr Gly Gly Lys Thr Cys Met Tyr
               100                 105                 110
   Gly Gly Ile Thr Lys His Glu Gly Asn His Phe Asp Asn Gly Asn Leu
           115                 120                 125
   Gln Asn Val Leu Ile Arg Val Tyr Glu Asn Lys Arg Asn Thr Ile Ser
       130                 135                 140
   Phe Glu Val Gln Thr Asp Lys Lys Ser Val Thr Ala Gln Glu Leu Asp
   145                 150                 155                 160
   Ile Lys Ala Arg Asn Phe Leu Ile Asn Lys Lys Asn Leu Tyr Glu Phe
                   165                 170                 175
   Asn Ser Ser Pro Tyr Glu Thr Gly Tyr Ile Lys Phe Ile Glu Asn Asn
               180                 185                 190
   Gly Asn Thr Phe Gln Tyr Asp Met Met Pro Ala Pro Gly Asp Lys Phe
           195                 200                 205
   Asp Gln Ser Lys Tyr Leu Met Met Tyr Asn Asp Asn Lys Thr Val Asp
       210                 215                 220
   Ser Lys Arg Val Lys Ile Glu Val His Leu Thr Thr Lys Asn Gly Xaa
   225                 230                 235                 240
<210> 9
   <211> 11
   <212> PRT
   <213> Staphylococcus aureus
<400> 9
   Cys Ala Gly Gly Thr Pro Asn Lys Thr Ala Cys
   1 5 10
<210> 10
   <211> 21
   <212> PRT
   <213> Staphylococcus aureus
<400> 10
   Cys Tyr Phe Ser Lys Lys Thr Asn Asp Ile Asn Ser His Gln Thr Pro
   1 5 10 15
   Lys Arg Lys Thr Cys
   20
<210> 11
   <211> 18
   <212> PRT
   <213> Staphylococcus aureus
<400> 11
   Cys Tyr Phe Ser Ser Lys Asp Asn Val Gly Lys Val Thr Gly Gly Lys
   1 5 10 15
   Thr Cys
<210> 12
   <211> 18
   <212> PRT
   <213> Staphylococcus aureus
<400> 12
   Cys Tyr Phe Ser Ser Lys Asp Asn Val Gly Lys Val Thr Gly Gly Lys
   1 5 10 15
   Thr Cys
<210> 13
   <211> 18
   <212> PRT
   <213> Staphylococcus aureus
<400> 13
   Cys Tyr Phe Ser Ser Lys Asp Asn Val Gly Lys Val Thr Gly Gly Lys
   1 5 10 15
   Thr Cys
<210> 14
   <211> 18
   <212> PRT
   <213> Staphylococcus aureus
<400> 14
   Cys Tyr Phe Ser Ser Lys Asp Asn Val Gly Lys Val Thr Ser Gly Lys
   1 5 10 15
   Thr Cys
<210> 15
   <211> 18
   <212> PRT
   <213> Staphylococcus aureus
<400> 15
   Cys Tyr Phe Ser Ser Lys Asp Asn Val Gly Lys Val Thr Gly Gly Lys
   1 5 10 15
   Thr Cys
<210> 16
   <211> 18
   <212> PRT
   <213> Staphylococcus aureus
<400> 16
   Cys Tyr Phe Ser Ser Lys Asp Asn Val Gly Lys Val Thr Gly Gly Lys
   1 5 10 15
   Thr Cys
<210> 17
   <211> 18
   <212> PRT
   <213> Staphylococcus aureus
<400> 17
   Cys Cys Phe Ser Ser Lys Asp Asn Val Gly Lys Val Thr Gly Gly Lys
   1 5 10 15
   Thr Cys
<210> 18
   <211> 11
   <212> PRT
   <213> Staphylococcus aureus
<400> 18
   Lys Tyr Lys Gly Lys Lys Val Asp Leu Tyr Gly
   1 5 10
<210> 19
   <211> 11
   <212> PRT
   <213> Staphylococcus aureus
<400> 19
   Lys Tyr Lys Asp Lys Tyr Val Asp Val Phe Gly
   1 5 10
<210> 20
   <211> 11
   <212> PRT
   <213> Staphylococcus aureus
<400> 20
   Lys Tyr Lys Asp Glu Val Val Asp Val Tyr Gly
   1 5 10
<210> 21
   <211> 11
   <212> PRT
   <213> Staphylococcus aureus
<400> 21
   Lys Tyr Lys Asp Glu Val Val Asp Val Tyr Gly
   1 5 10
<210> 22
   <211> 11
   <212> PRT
   <213> Staphylococcus aureus
<400> 22
   Lys Tyr Lys Asp Glu Val Val Asp Val Tyr Gly
   1 5 10
<210> 23
   <211> 11
   <212> PRT
   <213> Staphylococcus aureus
<400> 23
   His Phe Lys Ser Lys Asn Val Asp Val Tyr Pro
   1 5 10
<210> 24
   <211> 11
   <212> PRT
   <213> Staphylococcus aureus
<400> 24
   Lys Tyr Lys Gly Lys Lys Val Asp Leu Tyr Gly
   1 5 10
<210> 25
   <211> 11
   <212> PRT
   <213> Streptococcus pyrogenes
<400> 25
   Leu Phe Lys Asp Lys Asn Val Asp Ile Tyr Gly
   1 5 10
<210> 26
   <211> 11
   <212> PRT
   <213> Streptococcus pyrogenes
<400> 26
   Phe Lys Arg Asp Asp His Val Asp Val Phe Gly
   1 5 10
<210> 27
   <211> 11
   <212> PRT
   <213> Staphylococcus aureus
<400> 27
   Phe Thr Lys Gly Glu Lys Val Asp Leu Asn Thr
   1 5 10
<210> 28
   <211>17
   <212> PRT
   <213> Staphylococcus aureus
<400> 28
   Lys Lys Asn Val Thr Val Gln Glu Leu Asp Leu Gln Ala Arg Arg Tyr
   1 5 10 15
   Leu
<210> 29
   <211> 17
   <212> PRT
   <213> Staphylococcus aureus
<400> 29
   Lys Lys Lys Val Thr Ala Gln Glu Leu Asp Tyr Leu Thr Arg His Tyr
   1 5 10 15
   Leu
<210> 30
   <211> 17
   <212> PRT
   <213> Staphylococcus aureus
<400> 30
   Lys Lys Ser Val Thr Ala Gln Glu Leu Asp Ile Lys Ala Arg Asn Phe
   1 5 10 15
   Leu
<210> 31
   <211> 17
   <212> PRT
   <213> Staphylococcus aureus
<400> 31
   Lys Lys Ser Val Thr Ala Gln Glu Leu Asp Ile Lys Ala Arg Asn Phe
   1 5 10 15
   Leu
<210> 32
   <211> 17
   <212> PRT
   <213> Staphylococcus aureus
<400> 32
   Lys Lys Ser Val Thr Ala Gln Glu Leu Asp Ile Lys Ala Arg Asn Phe
   1 5 10 15
   Leu
<210> 33
   <211> 17
   <212> PRT
   <213> Staphylococcus aureus
<400> 33
   Lys Lys Asn Val Thr Val Gln Glu Leu Asp Ala Gln Ala Arg Arg Tyr
   1 5 10 15
   Leu
<210> 34
   <211> 17
   <212> PRT
   <213> Staphylococcus aureus
<400> 34
   Lys Lys Glu Val Thr Val Gln Glu Leu Asp Leu Gln Ala Arg His Tyr
   1 5 10 15
   Leu
<210> 35
   <211> 17
   <212> PRT
   <213> Streptococcus pyrogenes
<400> 35
   Lys Lys Met Val Thr Ala Gln Glu Leu Asp Tyr Lys Val Arg Lys Tyr
   1 5 10 15
   Leu
<210> 36
   <211> 17
   <212> PRT
   <213> Streptococcus pyrogenes
<400> 36
   Lys Asp Ile Val Thr Phe Gln Glu Ile Asp Phe Lys Ile Arg Lys Tyr
   1 5 10 15
   Leu
<210> 37
   <211> 5
   <212> PRT
   <213> Staphylococcus aureus
<400> 37
   Lys Lys Gln Leu Ile
   1 5
<210> 38
   <211> 9
   <212> PRT
   <213> Staphylococcus aureus
<400> 38
   Leu Asp Phe Glu Ile Arg His Gln Leu
   1 5
<210> 39
   <211> 12
   <212> PRT
   <213> Staphylococcus aureus
<400> 39
   Cys Met Tyr Gly Gly Val Ile Leu His Asp Asn Asn
   1 5 10
<210> 40
   <211> 12
   <212> PRT
   <213> Staphylococcus aureus
<400> 40
   Cys Met Tyr Gly Gly Val Thr Glu His Asn Gly Asn
   1 5 10
<210> 41
   <211> 12
   <212> PRT
   <213> Staphylococcus aureus
<400> 41
   Cys Met Tyr Gly Gly Ile Thr Lys His Glu Gly Asn
   1 5 10
<210> 42
   <211> 12
   <212> PRT
   <213> Staphylococcus aureus
<400> 42
   Cys Met Tyr Gly Gly Ile Thr Lys His Glu Gly Asn
   1 5 10
<210> 43
   <211> 12
   <212> PRT
   <213> Staphylococcus aureus
<400> 43
   Cys Met Tyr Gly Gly Ile Thr Lys His Glu Gly Asn
   1 5 10
<210> 44
   <211> 12
   <212> PRT
   <213> Staphylococcus aureus
<400> 44
   Cys Thr Tyr Gly Gly Val Thr Pro His Glu Gly Asn
   1 5 10
<210> 45
   <211> 12
   <212> PRT
   <213> Staphylococcus aureus
<400> 45
   Cys Met Tyr Gly Gly Val Thr Leu His Asp Asn Asn
   1 5 10
<210> 46
   <211> 12
   <212> PRT
   <213> Streptococcus pyrogenes
<400> 46
   Cys Ile Tyr Gly Gly Val Thr Asn His Glu Gly Asn
   1 5 10
<210> 47
   <211> 12
   <212> PRT
   <213> Streptococcus pyrogenes
<400> 47
   Tyr Ile Tyr Gly Gly Ile Thr Pro Ala Gln Asn Asn
   1 5 10
<210> 48
   <211> 12
   <212> PRT
   <213> Staphylococcus aureus
<400> 48
   Phe Gln Ile Ser Gly Val Thr Asn Thr Glu Lys Leu
   1 5 10
<210> 49
   <211> 14
   <212> PRT
   <213> Staphylococcus aureus
<400> 49
   Leu Leu Arg Ile Tyr Arg Asp Asn Lys Thr Ile Asn Ser Glu
   1 5 10
<210> 50
   <211> 14
   <212> PRT
   <213> Staphylococcus aureus
<400> 50
   Tyr Leu Met Met Tyr Asn Asp Asn Lys Met Val Asp Ser Lys
   1 5 10
<210> 51
   <211> 14
   <212> PRT
   <213> Staphylococcus aureus
<400> 51
   Tyr Leu Met Met Tyr Asn Asp Asn Lys Thr Val Asp Ser Lys
    1               5                  10
<210> 52
   <211> 14
   <212> PRT
   <213> Staphylococcus aureus
<400> 52
   Tyr Leu Met Met Tyr Asn Asp Asn Lys Thr Val Asp Ser Lys
    1               5                  10
<210> 53
   <211> 14
   <212> PRT
   <213> Staphylococcus aureus
<400> 53
   Tyr Leu Met Ile Tyr Lys Asp Asn Lys Met Val Asp Ser Lys
    1               5                  10
<210> 54
   <211> 14
   <212> PRT
   <213> Staphylococcus aureus
<400> 54
   Gln Leu Arg Ile Tyr Ser Asp Asn Lys Thr Leu Ser Thr Glu
    1               5                  10
<210> 55
   <211> 14
   <212> PRT
   <213> Staphylococcus aureus
<400> 55
   Leu Leu Arg Ile Tyr Arg Asp Asn Lys Thr Ile Asn Ser Glu
   1 5 10
<210> 56
   <211> 14
   <212> PRT
   <213> Streptococcus pyrogenes
<400> 56
   Tyr Leu Met Ile Tyr Lys Asp Met Glu Thr Leu Asp Ser Asn
   1 5 10
<210> 57
   <211> 14
   <212> PRT
   <213> Streptococcus pyrogenes
<400> 57
   Ile Phe Ala Lys Tyr Lys Asp Asn Arg Ile Ile Asn Met Lys
   1 5 10
<210> 58
   <211> 14
   <212> PRT
   <213> Staphylococcus aureus
<400> 58
   Pro Pro Ile Asn Ile Asp Glu Ile Lys Thr Ile Glu Ala Glu
   1 5 10
<210> 59
   <211> 54
   <212> DNA
   <213> Staphylococcus aureus
<400> 59
   tgctattttt catccaaaga taatgtaggt aaagttacag gtggcaaaac ttgt 54
<210> 60
   <211> 18
   <212> PRT
   <213> Staphylococcus aureus
<400> 60
   Cys Tyr Phe Ser Ser Lys Asp Asn Val Gly Lys Val Thr Gly Gly Lys
   1 5 10 15
   Thr Cys
<210> 61
   <211> 42
   <212> DNA
   <213> Synthetic
<400> 61
   tgctattttt catccaaaga taatgcaggt ggcaaaactt gt 42
<210> 62
   <211> 14
   <212> PRT
   <213> Synthetic
<400> 62
   Cys Tyr Phe Ser Ser Lys Asp Asn Ala Gly Gly Lys Thr Cys
   1 5 10
<210> 63
   <211> 27
   <212> DNA
   <213> Synthetic
<400> 63
   tgctattttt catccggcaa aacttgt 27
<210> 64
   <211> 9
   <212> PRT
   <213> Synthetic
<400> 64
   Cys Tyr Phe Ser Ser Gly Lys Thr Cys
   1 5
<210> 65
   <211> 18
   <212> DNA
   <213> Synthetic
<400> 65
   tgctgtggca aaacttgt 18
<210> 66
   <211> 6
   <212> PRT
   <213> Synthetic
<400> 66
   Cys Cys Gly Lys Thr Cys
   1 5

## Description

### BACKGROUND OF THE INVENTION

A group of biological agents termed superantigens has been described based on their ability to stimulate monocytes and unprimed CD4⁺ (MHC Class II restricted) and CD8⁺ (MHC Class I restricted) T-cells. Included with the designation of superantigens are the enterotoxins of *Staphylococcus aureus.*

The enterotoxins of *Staphylococcus aureus* form a group of serologically distinct proteins, originally designated A, B, C₁, C₂, C₃, D, E, G, and H. Subsequently, a number of variants have been described. These proteins and toxic shock syndrome proteins were originally recognized as the causative agents of staphylococcal food poisoning. Ingestion of preformed enterotoxin in contaminated food leads to the rapid development (within two to six hours) of symptoms of vomiting and diarrhea that are characteristic of staphylococcal food poisoning. Toxic shock syndrome toxin-1, TSST-1, a distantly related protein also produced by *S. aureus,* is classically responsible for the toxic shock syndrome, although other staphylococcal enterotoxins may result in the syndrome due to the induction of cytokines.

Other agents which have been identified as superantigens include for example, the staphylococcal exfoliative toxins, A and B; the mammary tumor virus superantigen; rabies virus nucleocapsid protein; pyrogenic exotoxins A, B, C from *S. pyrogens;* and the *Mycoplasma arthritides* mitogen. Additional biological agents which have been demonstrated to have superantigen properties include the human immunodeficiency virus (HIV), gp120 and peptides from HIV, mouse mammary tumor virus and feline immunodeficiency virus. A Leishmania peptide antigen has also been disclosed as a superantigen.

Superantigens, unlike conventional antigens, do not require processing *in-vivo.* In general, superantigens have two binding regions, one of which interacts with the Class II major histocompatibility complex (MHC) on the antigen presenting cell and the other which interacts with the Vβ variable region of the T-cell receptor on CD4 and/or CD8 cells. Various enterotoxins bind to one or more of the different Vβ receptor epitopes. In contrast to conventional antigens, superantigens do not occupy the T-cells receptor cleft but are felt to bind to an external region thus explaining the ability to activate a broad population of T-cells.

Enterotoxins produced by *Staphylococcus aureus* include a group of related proteins of about 20 to 30 Kd. The complete amino acid composition of a number of staphylococcal enterotoxins and streptococcal pyrogenic exotoxin has been reported (see e.g., PCT Patent Appl. No. WO 93/24136.)

Staphylococcal enterotoxins ("SEs") were initially classified on the basis of their antigenic properties into groups A, B, C1, C2, C3, D, and E. Subsequent relatedness was based on peptide and DNA sequence data. Among the staphylococcal enterotoxins, groups B and C are closely related and groups A, D, and E are closely related in amino acid sequence. SEC1, SEC2, and SEC3 and related isolates share approximately 95% sequence similarity. Table 1 shows the alignment of the predicted sequences of the eight known SEC variants following cleavage of the signal peptide. The N-terminus of each of the mature proteins was verified by amino acid sequencing. Amino acid positions that contain residues that are not conserved among all SEC are indicated by asterisks. SEB and SEC are approximately 45-50% homologous. In contrast, non-enterotoxin superantigens, TSST-1 and *Streptococcal Pyrogenic Enterotoxin* C (SPEC) share only approximately 20% primary sequence homology to SEC. Despite these differences, the tertiary structure of the various enterotoxins show nearly identical folds.

The staphylococcal enterotoxins A, B, C₁, C₂, C₃, D, E, G and H share a common structural feature of a disulfide bond not present in other enterotoxins. Table 2 shows the position of the disulfide bond in a number of enterotoxins. Data in reference to the active sites of the enterotoxin molecule in relationship to biological activity, MHC binding, and TCR binding has been obtained. Sequence data demonstrate a high degree of similarity in four regions of the enterotoxins (See Table 3). The peptides implicated in potential receptor binding correspond to regions 1 and 3 which form a groove in the molecule. Amino acid residues within and adjacent to the α₃ cavity of SEC3 have been shown to relate to T-cell activation.

**Table 2. LOCATION OF DISULFIDE LOOP IN STAPHYLOCOCCUS ENTEROTOXINS**

| ENTEROTOXIN | AMINO ACID RESIDUES | AMINO ACID SEQUENCE OF DISULFIDE LOOP |
|---|---|---|
| SEA | 96-106 | 96→CAGGTPNKTAC |
| SEB | 93-114 | 93→CYFSKKTNDINSHQTPKRKTC |
| SEC1 | 93-110 | 93→CYFSSKDNVGKVTGGKTC |
| SEC2 | 93-110 | 93→CYFSSKDNVGKVTGGKTC |
| SEC3 FRI 913 | 93-110 | 93→CYFSSKDNVGKVTGGKTC |
| SEC3 FRI 909 | 93-110 | 93→CYFSSKDNVGKVTSGKTC |
| SEC 4446 | 93-110 | 93→CYFSSKDNVGKVTGGKTC |
| SEC-Bovine | 93-110 | 93→CYFSSKDNVGKVTGGKTC |
| SEC-Ovine | 93-110 | 93→CCFSSKDNVGKVTGGKTC |

The staphylococcal enterotoxins are potent activators of T-cells, resulting in proliferation and the generation of cytotoxic T-cells. SEA is a potent T-cell mitogen eliciting strong polyclonal activation at concentrations of 10⁻¹³ to 10⁻¹⁰ molar in human systems.

The staphylococcal enterotoxins, aside from the acute gastroenteritis and toxic shock syndrome associated with them, have been shown to have a variety of other beneficial biological effects. The biological effects of these agents and the toxic shock syndrome are due in part to the ability of staphylococcal enterotoxins to induce cytokines. Various cytokines described include IL-1, IL-2, and tumor necrosis factor ("TNF"). More recently SEB and toxic shock syndrome toxin ("TSST-1") have been shown to induce interleukin-12, an inducer of cell mediated immunity, in human peripheral blood mononuclear cells. (See Leung et al., *J Exp Med,* 181:747 (1995)). The antitumor activity of treating cancer in rabbits utilizing 40 to 60 µg/kg of a staphylococcal enterotoxin has been disclosed in PCT Patent Appl. Nos. WO 91/10680 and WO 93/24136.

Exposure to enterotoxin either *in-vitro* or *in-vivo* leads to depletion of T-cells having the appropriate Vβ receptor through programmed cell death in some strains of mice, specifically Balb/c and CBA/2. Cell death can be prevented by high doses of retinol or RU-38486. Programmed cell death has not been observed upon exposure of human cells to enterotoxins.

Although the systemic lethal toxicity of enterotoxins has been related to their ability to induce cytokines, particularly IL-1, IL-2 and gamma interferon, lethal toxicity also appears to be related to a synergistic activity with endogenous endotoxins and the ability of the liver to detoxify endotoxins. Although a number of animals have been utilized to evaluate lethality, the accepted model is the continous infusion over a period of time, usually 4 days, in rabbits. The direct toxic dose varies among various species. The 50% lethal dose of TSST-1 is approximately 50 µg/kg for Balb/c mice. Piglets, although showing clinical manifestations of toxic shock syndrome, tolerate doses of 100 µg/kg of TSST-1. TSST-ovine is known to be non-toxic at doses of 200 µg in rabbits.

In Dutch belted rabbits, intramuscular injection of 50 mg/kg of staphylococcal enterotoxin B caused death. Intravenous injection at 0.5 to 1.0 mg/kg of enterotoxin A or B in rhesus macaques results in hypotension and death (Liu C.T., et al *Amer J Vet Res* 39:279 and 1213, 1978).

In contrast to other species, man is extremely sensitive to enterotoxins. One (1) mg of TSST-1, approximately 15 nanogram/kg, can be lethal for man. Therefore, the recommended doses currently proposed in the art for treating man are unacceptable. There is a need, therefore, for mutant staphylococcal enterotoxins which are non-toxic at anticipated doses for man while still retaining desirable biological activity.

Several studies of staphylococcal enterotoxin have identified a number of biologically active modified or mutant enterotoxins with reduced toxicity. Carboxymethylation of SEB results in a loss of gastrointestinal toxicity but not mitogenic activity. Studies with the TSST-1 have demonstrated the active site to be between amino acids residue 115 and 141. Point mutation of site 135 from histidine to alanine results in a loss of mitogenic activity and toxicity (See Bonventre P.F., et al. *Infect Immun* 63:509 (1995)). Studies with the staphylococcal enterotoxin SEC1 demonstrated that the disulfide bond between residue 93 and 110 is not required for activity (See Hovde et al., *Mol Microbiol* 13:897 (1994)). Studies of the molecular binding region of staphylococcal enterotoxin B using overlapping peptides demonstrated peptide 124 to 154 inhibited SEB induced mitogenic activity.

Based on the known biological activities of the toxic native enterotoxins, it is desirable to create mutants which are at least 1000-fold or more less toxic compared to native enterotoxins and retain biological activity. Recent studies have demonstrated that mutant enterotoxins can be produced which retain certain biological activities and which may be significantly less lethal as determined in rabbits. A mutant of the TSST-1 enterotoxin which differs in amino acid 136 and is non-lethal at ten times the lethal dose of the native toxin (in rabbits), but retains biological activity has been disclosed. A number of mutants of SEC1, unable to form a disulfide bond, have been reported to be ten times less toxic than the native toxin while retaining biological activity. (See e.g., Hovde et al., *Molec Microbiol* 13:897 (1994)).

### SUMMARY OF THE INVENTION

The present invention relates to modified versions of disulfide loop-containing bacterial pyrogenic toxins. The modified pyrogenic toxins retain useful biological properties but have substantially reduced toxicity (e.g., toxicity reduced by at least about 10-fold) compared to the corresponding unmodified native toxin. Selected deletions within the disulfide loop region can produce modified toxins having a 100-fold or greater decrease in toxicity. The toxicity of the modified toxin can be measured based on a variety of parameters, including emetic response inducing activity, fever inducing activity, and lethality (as measured by LD₅₀ in Dutch Belted rabbits).

Examples of the present modified toxins include disulfide loop region deletion mutants of native toxins derived from *Staphylococcus aureus* or *Streptococcus pyrogenes.* Suitable native disulfide loop-containing toxins which may be modified according to the present invention include Type A, B, C, D and E staphylococcal enterotoxins as well as streptococcal pyrogenic enterotoxin A ("SPEA") and streptococcal superantigen ("SSA") produced by *S. pyrogenes.*

The pyrogenic toxins constitute a family of exotoxins produced by species of gram positive cocci, such as Staphylococcus and Streptococcus. The pyrogenic toxins are characterized by shared ability to induce fever, enhance host susceptibility to endotoxin shock, and induce T cell proliferation through action as superantigens. Examples of pyrogenic toxins include TSST-1, staphylococcal enterotoxins (SEs), and streptococcal pyrogenic exotoxins (SPEs). In addition to the activities listed above, some pyrogenic toxins have additional activities that are not shared by all pyrogenic toxins. For example, the staphylococcal enterotoxins induce emesis and diarrhea when ingested. Structurally, the pyrogenic toxins have varying degrees of relatedness at the amino acid and nucleotide sequence levels. A number of the pyrogenic toxins include a disulfide loop as a structural feature. The staphylococcal enterotoxins have a disulfide loop, as do some others in this family. Examples of other pyrogenic toxins that have a disulfide loop are the streptococcal superantigen ("SSA") and streptococcal pyrogenic exotoxin A ("SPEA").

The pyrogenic toxins have varying degrees of relatedness which provides the basis for separating some of them informally into subgroups. One subgroup includes staphylococcal type B and C enterotoxins ("SEB" and "SEC"), as well as SPEA and SSA. These toxins share between about 49% to greater than 95% amino acid sequence homology (Reda et al, Infect. Immun., 62:1867-1874: (1994)). Another subgroup of related pyrogenic toxins include staphylococcal type A and E enterotoxins (SEA and SEE) which are 83% homologous to each other (Couch et al, J. Bacteriol.,170:2954- 60 (1988), less so but significantly to SED (Bayles et al., J. Bacteriol., 171:4799-4806 (1989)). The amino acid sequences of this second subgroup is more distantly related to SEB, SEC, SPEA, and SSA. Examples of pyrogenic toxins having disulfide bonds are present in both of these two subgroups. TSST-1 and streptococcal pyrogenic exotoxins B and C (SPEB and SPEC) are examples of a third subgroup of less related toxins. Although toxins from this third subgroup may share some conserved regions (see table 3) with toxins from the other subgroups, there is little overall sequence homology between toxins in the third subgroup and the pyrogenic toxins in the other two subgroups. Neither TSST-1, SPEB nor SPEC includes a disulfide loop.

The disulfide loop region of a native pyrogenic toxin, such as a native staphylococcal enterotoxin, is generally modified through deletion of a number of amino acid residues within the loop. The modification typically includes deletion of amino acid residues within the disulfide loop region and may include one or more substitutions and/or additions to the remaining loop residues. After modification, the disulfide loop region typically contains no more than about 10 and, preferably, no more than about 6 amino acids residues. In another embodiment of the invention, a modified pyrogenic toxin is formed from a native pyrogenic toxin modified by deletion of at least 40% of the amino acid residues within the disulfide loop region, e.g., by deletion of 8 or more amino acid residues from the disulfide loop region of a native type C staphylococcal enterotoxin.

The present invention is also directed to isolated nucleic acids which include a nucleotide sequence encoding a modified pyrogenic toxin.

### DETAILED DESCRIPTION OF THE INVENTION

The current invention relates to modified versions of pyrogenic toxins, such as staphylococcal enterotoxins produced by modifications which include deletions in the disulfide bond region. The present invention describes the feasibility of obtaining mutant pyrogenic toxins which retain biological activity but demonstrate significantly lower toxicity at doses well in excess of the normal lethal dose and levels anticipated for human therapeutic use.
Therefore the invention provides a pyrogenic toxin derived from a native disulfide loop-containing pyrogenic toxin, wherein the toxin derived from a native disulfide loop-containing pyrogenic toxin is called modified toxin and comprises a disulfide loop region containing no more than 10 amino acid residues. Further specified pyrogenic toxins of the present invention are described in claims 2-20, 23 and 24. It is further provided an isolated nucleic acid comprising a nucleotide sequence encoding a pyrogenic toxin and a staphylococcal enterotoxin, respectively, as lined out in claims 21 and 22.

The modified pyrogenic toxins are derived from a native pyrogenic toxin having a disulfide loop. The terms "disulfide loop" and "disulfide loop region" are used interchangeably herein. As employed in this application, these terms refer to the sequence of about 10 to about 30 amino acid residues forming a loop defined by a disulfide bond in a native pyrogenic toxin. The term "disulfide loop region" also refers to the corresponding portion of the sequence of a modified pyrogenic toxin which has been produced by deletion, substitution or addition of one or more amino acid residues of the disulfide loop of a native pyrogenic toxin. The disulfide loop region is defined to begin with the N-terminal Cys residue and end with the C-terminal Cys residue of the loop, e.g., amino acid residues 93-110 of staphylococcal enterotoxin C1. As used herein, the positions of the disulfide loop region for a given native pyrogenic toxin are numbered beginning with the N-terminal cyteine residue in the loop, e.g., position 93 of type B or C staphylococcal enterotoxins is also referred to herein as position 1 of the disulfide loop region.

The modification of the disulfide loop typically includes deletion of at least about 40% of the amino acid residues within the disulfide loop. For example, this generally results in the deletion of at least about 8 amino acid residues from the disulfide loop region of an SEC. Examples of native staphylococcal enterotoxin which can be modified to form the present low toxicity toxins include type A, B, C, D, E, G, and H staphylococcal enterotoxins. Type C staphylococcal enterotoxins such as staphylococcal enterotoxin C1, staphylococcal enterotoxin C2, staphylococcal enterotoxin C2, staphylococcal enterotoxin C-MNCopeland, staphylococcal enterotoxin C-4446, staphylococcal enterotoxin C-bovine (GenBank Accession No. L13374), staphylococcal enterotoxin C-canine (GenBank Accession No. V 19526) and staphylococcal enterotoxin C-ovine (GenBank Accession No. L13379) are particularly suitable enterotoxins for modification by deletion of a portion of the disulfide loop region to form a staphylococcal enterotoxin with decreased toxicity.

The modified disulfide loop region generally contains no more than about 10 amino acid residues and, preferably no more than 6 amino acid residues. For example, a type C staphylococcal enterotoxin, such as staphylococcal enterotoxin C1, can be modified to delete amino acid residues 98-106 (residues 6-14 of the disulfide loop) to form a modified staphylococcal enterotoxin having substantially reduced toxicity. An even greater reduction in toxicity is produced by deleting amino acid residues 95-106 (disulfide loop residues 3-14) of staphylococcal enterotoxin C1. Both of these mutants, despite having substantially reduced toxicity, are biologically active as evidenced by their ability to stimulate the uptake of thymidine by human peripheral blood mononuclear cells.

In addition to deletion of a number of the disulfide loop residues, substitution of a cysteine residue for the residue at position 2 of disulfide loop can contribute to a decrease in the toxicity of a pyrogenic toxin such as a type C staphylococcal enterotoxin. In a preferred embodiment, staphylococcal enterotoxin C1 can be modified so that in addition to deletion of a substantial portion of the residues in the loop region, the amino acid residue at position 2 of the loop, Tyr-94, is replaced by a cysteine.

Other examples of preferred embodiments of the invention include modified staphylococcal enterotoxins having no more than 10 amino acid residues and, preferably, no more than 6 amino acid residues in the disulfide loop region. Particularly preferred examples include staphylococcal enterotoxins with no more than about 9 amino acid residues which include the sequence Cys-Gly-Lys-Thr. One example of such a mutant is staphylococcal enterotoxin C1 modified to have the disulfide loop region sequence Cys-Cys-Gly-Lys-Thr-Cys.

The methods of this invention employed in preparing mutant enterotoxins, and their screening, analysis, and purification are known in the art and described herein. Site directed mutagenesis can be carried out by initially cloning the SEC1 structural gene *SEC*_{*MNDON*} on a 1.4 Kb MindITI-BamHI restriction fragment. A unique *SphI* restriction site (5'-GCATGC-3') can be introduced into *SEC*_{*MNDON*} in the region coding for the disulfide loop at nucleotides 301-306 (5'-GTAGGT-3') using a commercially available kit (Altered Sites *in vitro* Mutagenesis System, Promega). Potential mutants may be screened by *Sph1* digestion and confirmed by nucleic acid sequence analysis.

Select *SEC*_{*MNDON*} deletion mutants were cloned into an *E. coli* cell line using pMIN164 an *E. coli - S. aureus* shuttle vector. To facilitate protein purification, recombinant plasmids from *E. coli* RR1 transformants were transformed into *S*. *aureus* RN4220 by standard protoplast transformation techniques. Plasmids containing cloned toxin genes were maintained in *S. aureus* RN4220 under erythromycin (50 mg/ml) selection. For purification of native and mutant derivatives of the toxin recombinants, *S. aureus* RN4220 cultures were grown in dialyzable beef heart media supplemented with 1% glucose buffer (330 mM glucose; 475 mM NaHCO₃; 680 mM NaCl; 137 mM Na₂HPO₄.H₂O; 28 mM L-glutamine) and erythromycin (50 mg/ml) followed by ethanol precipitation.

Purification of ethanol precipitated proteins was accomplished by preparative flat bed isoelectric focusing (IEF). Following the IEF run, proteins in select fractions were pooled, dialyzed to remove the amphylotes and then visually assessed by sodium dodecyl sulfate polyacrylamide gel electrophoresis (SDS-PAGE).

The toxicity and biological activity of the mutants was evaluated using standard methods known to those skilled in the art as described below. The emetic activity of SEC1, and the SEC1 mutant toxins was determined by a modification of the standard monkey feeding assay using young adult pigtail monkeys (*M. nemestrina*). The animals were manually restrained while the toxin was administered through a nasogastric tube (Infant feeding tube; Becton Dickinson, Rutherford, NJ). Toxins were screened for retention of emetic activity at a dose of 10 µg/Kg which is approximately 100 times the minimal emetic dose for SEC1. Non-emetic toxins were tested for residual emetic activity at a high dose of 250 µg/Kg.

The mitogenic capacities of mutant toxins were compared to that of SEC1 native toxin by using human peripheral blood mononuclear cells (PMBC) in a standard 4-day assay. Solutions of native and mutant toxin were added in triplicate to PBMC cell suspensions, 1x10⁶ cells/ml, in 96-well tissue culture plates. This mixture of cells and toxin was then incubated at 37°C under atmospheric conditions of 6% CO₂ for 72 hours. [³H]-thymidine (New Research Products, Boston, MA) at a concentration of 1 µCi/25 µl in a complete RPMI medium was added to each well and allowed to incubate under the same conditions for an additional 18-24 hours. After incubation, radiolabeled cellular nucleic acids were harvested onto glass fiber filters (Skatron, Sterling, VA) using a semi-automatic cell harvester (Skatron). Lymphocyte proliferation was quantitated by measuring incorporation of [³H]-thymidine into cellular DNA using a liquid scintillation counter (TRI-CARB 1500 Liquid Scintillation Center, Packard, Rockville, MD).

The ability of a staphylococcal enterotoxin to induce a fever response and enhance susceptibility of lethal endotoxic shock can be determined *in vivo* using a standard rabbit model (Bohach et al., Infect. Immun., 55, 428 (1987)). Following conditioning in a test rack and having baseline body temperature recorded, adult New Zealand White rabbits can be initially intravenously injected with a native or mutant toxin at a concentration of 10 µg/kg in sterile physiological saline. Sterile saline and purified SEC1 toxin are typically used as negative and positive controls respectively. Following toxin injection, rabbit body temperature is generally monitored rectally every hour for four hours. Four hours after initial treatment, an intravenous injection of lipopolysaccharide ("LPS") from *Salmonella typhimurium* (Difco Laboratories, Detroit, Michigan) is administered intravenously at a concentration of 10 µg/Kg in sterile saline. Animals are then observed for signs of shock and mortality for 48 hours after LPS injection.

Cytokine induction may be determined by utilizing isolated mononuclear cells from heparinized venous blood. Briefly, heparinized venous blood is obtained and layered onto lymphocyte separation medium. The tubes are spun and the mononuclear layer is harvested, washed in PBS, resuspended in RPMI containing 10% FCS and adjusted to 1 x 10⁶ cells per ml. Aliquots of 100 µl are typically placed into 96-well microtiter plates. The enterotoxins were added in 100 µl to a final concentration of 1 ng/ml. Following incubation at 37°C for 48-72 hours, the supernatant was harvested can be assayed for cytokines using commercially available kits from R&D Systems Minneapolis, MN.

The invention will be further described by reference to the following examples. These examples illustrate but do not limit the scope of the invention that has been set forth herein. Variation within the concepts of the invention will be apparent.

### EXAMPLE 1

The SEC1 structural gene, *SEC*_{*MNDON*}*,* was previously cloned on a 1.4 Kb *HindIII-BamH1* restriction fragment (Bohach et al., Infect. Immun., 55, 428 (1987)). A unique *SphI* restriction site (5'-GCATGC-3') was introduced into *SEC*_{*MNDON*} in the region coding for the disulfide loop at nucleotides 301-306 (5'-GTAGGT-3') using a commercially available kit (Altered Sites *in vitro* Mutagenesis System, Promega). Potential mutants were screened by *Sph1* digestion and confirmed by nucleic acid sequence analysis.

### EXAMPLE 2

The unique *Sph1* site was used to linearize the *SEC*_{*MNDON*} gene so that bi-directional deletions could be generated using *Bal-31* exonuclease (Boehringer Mannheim, Indianapolis, IN). *Bal-31* generated deletion mutant plasmids were ligated and transformed into *E. coli* TG1. Transformants growing on LB-AMP (125 µg/ml) with in-frame stable deletions were detected by screening with SEC1 specific rabbit antisera in immunodiffusion and analyzed by nucleic acid sequence analysis. Sequencing reactions were done using *Sequenase Version 2.0,* a commercially available kit (U.S. Biochemical Corp., Cleveland, Ohio).

Three deletion mutants were chosen for detailed analysis based on the size and location of the deletions in the loop region of the toxin; SEC1-4AA, SCE1-9AA, and SEC1-12AA. Following purification, each of the disulfide loop mutant toxins could be distinguished from wild type SEC1 on the basis of size when analyzed by sodium dodecyl sulfate polyacrylamide gel electrophoresis.

The nucleic acid and amino acid sequences for SEC1 and three deletion mutants are shown in Table 4.

### EXAMPLE 3

### Mutant Toxin Purification

Select *SEC*_{*MNDON*} deletion mutants were cloned into E. *coli* RR1 using pMIN164, an *E*. *coli - S. aureus* shuttle vector. To facilitate protein purification, recombinant plasmids from E. *coli* RR1 transformants were transformed into *S. aureus* RN4220 by standard protoplast transformation techniques. Plasmids containing cloned toxin genes were maintained in *S. aureus* RN4220 under erythromycin (50 µg/ml) selection. For purification of the recombinantly produced native and mutant derivatives of the toxin, *S. aureus* RN4220 cultures were grown in dialyzable beef heart media supplemented with 1% glucose buffer (330 mM glucose; 475 mM NaHCO₃; 680 mM NaCl; 137 mM Na₂HPO₄.H₂O 28 mM L-glutamine) and erythromycin (50 µg/ml) followed by ethanol precipitation.

Purification of ethanol precipitated proteins was accomplished by preparative flat bed isoelectric focusing ("IEF"). Following the IEF run, proteins in select fractions were pooled, dialyzed to remove the ampholytes and then visually assessed by sodium dodecyl sulfate polyacrylamide gel electrophoresis (SDS-PAGE).

### EXAMPLE 4

### Emesis Assay

Assays of the emetic activity of SEC and the modified SEC1 toxins were conducted using a modification of the standard monkey feeding assay using young adult pigtail monkeys (*M. nemestrina*). The animals were manually restrained while the toxin was administered through a nasogastric tube (Infant feeding tube; Becton Dickinson, Rutherford, NJ). Toxins were screened for retention of emetic activity at a dose of 10 µg/Kg which is approximately 100 times the minimal emetic dose for SEC1. Non-emetic toxins were tested for residual emetic activity at a high dose of 250 mg/Kg.

It has been previously shown that the minimal emetic dose of wild type SEC1 for *M. nemestrina* was 0.1 µg/Kg. For initial experiments in which the emetic ability was tested, loop mutant toxins were administered at 10 µg/Kg. This insured an excess of toxin over the wild type SEC1 minimal emetic dose. Following intragastric toxin inhibition, animals were observed for at least 12 hours for an emesis response. SEC1-12AA did not show emesis at the 10 µg/Kg concentration and was subsequently tested for emesis at a higher toxin concentration, 250 µg/Kg. Even at this higher doseage level, the SEC1-12AA loop mutant toxin showed no emetic response (See Table 5).

**Table 5. Emetic Response^{c} of SEC1 Loop Deletion Mutants**

| **Dose**^{**a**} | **SEC1** | **SEC1-4aa** | **SEC1-9aa** | **SEC1-12aa** |
|---|---|---|---|---|
| 250 µg/Kg | ND^{b} | ND | ND | 0/2 |
| 10 µg/Kg | 2/2 | 2/2 | 2/2 | 0/2 |
| 1 µg/Kg | 2/2 | 2/2 | 0/1 | ND |

| | | | | |
|---|---|---|---|---|
| ^{a}µg Toxin/Kg Body Weight | | | | |
| ^{b} ND = Not Determined | | | | |
| ^{c} Number animals exhibiting emetic response/Total number of animals | | | | |

### EXAMPLE 5

### Pyrogenicity and Enhancement of Lethal Endotoxic Shock

The SEC 1 mutant toxin's ability to induce a fever response and enhance susceptibility of lethal endotoxic shock was determined *in vivo* using a previously described rabbit model. Following 1 hour of conditioning in a test rack and having baseline body temperature recorded, adult New Zealand White rabbits were initially intravenously injected with SEC1 or SEC1 mutant toxin at a concentration of 10 µg/Kg in sterile physiological saline. Sterile saline and purified SEC1 toxin were used as negative and positive controls, respectively. Following toxin injection, rabbit body temperature was monitored rectally every hour for four hours. Four hours after initial treatment an intravenous injection of lipopolysaccharide (LPS) from *Salmonella typhimurium* (Difco Laboratories, Detroit, Michigan) was administered intravenously at a concentration of 10 µg/Kg in sterile saline. Animals were watched for signs of shock and mortality for 48 hours after LPS injection.

Wild type SEC1, administered at both 10 µg/Kg and 1 µg/Kg, showed a typical temperature rise (Table 6) as well as an enhanced susceptibility to endotoxic shock (Table 7).

**Table 6. Maximum Temperature Rise in °C in Rabbits Given Noted Amount of Native SEC1, Compound to SEC1-4, SEC1-9, or SEC1-12 Mutant**

| | **SEC1** | **SEC1-4aa** | **SEC1-9aa** | **SEC1-12aa** |
|---|---|---|---|---|
| 100 µg | ND | ND | ND | 0.6 |
| 10 µg | 1.6 | 1.46 | 0.9 | 0.45 |
| 1 µg | 1.3 | 1 | 0.43 | ND |
| 0.1 µg | 1.05 | 0.65 | ND | ND |
| 0.01 µg | 0.475 | 0.425 | ND | ND |

**Table 7. Lethality in Dutch Belted Rabbits of Native SEC1 and SEC1 Deletion Mutants**

| **Dose (µg)** | **SEC1** | **SEC1-4** | **SEC1-9** | **SEC1-12** |
|---|---|---|---|---|
| 100 | ND | ND | ND | 0/3 |
| 10 | 3/3 | 3/3 | 2/3 | 0/3 |
| 1 | 2/2 | 3/3 | 0/3 | ND |
| 0.1 | 3/3 | 2/3 | ND | ND |
| 0.01 | 1/4 | 0/3 | ND | ND |

SEC1-12AA loop mutant toxin concentration was increased to 100 µg/Kg after test animals showed both reduced pyrogenic effects and no susceptibility to endotoxin shock at the initial dose tested, 10 µg/Kg. Following this log fold increase of the SEC-12AA mutant toxin to 100 µg/Kg it was seen that the mutant toxin induced a slight temperature increase over the initial SEC1-12AA loop mutant toxin concentration tested but was not lethal in the assay.

### EXAMPLE 6

### Mitogenicity

The mitogenic capacity of mutant toxin was compared to that of SEC1 native toxin by using human peripheral blood mononuclear cells (PMBC) in a standard 4-day assay (Bohach et al., Infect. Immun., 55, 428 (1987)). Human peripheral blood mononuclear cells were isolated from 30 to 60 ml of heparinized blood obtained by venipuncture. The blood was mixed 1:1 with Dulbecco's Phosphate Buffered Saline (PBS) (without Calcium or magnesium) and layered onto Fico/Lite (density 1.079 g/ml, Atlanta Biologicals, Norcross, GA) and centrifuged at 400 x g for 20 minutes. The interface containing the mononuclear cells was removed and washed 3 times with PBS. After the last wash, the pellet was resuspended in Hank's Balanced Salt solution without calcium or magnesium and layered on Fetal Bovine Serum (Atlanta Biologicals, Norcross, GA) and centrifuged at 100 x g for 10 minutes to remove the platelets. The cells were washed in Hank's Balanced Salt solution, Cat. No. M1211-021-LV (Atasca, IL). A cell count was done by Trypan Blue exclusion.

Solutions of native and mutant toxins were added in triplicate to PMBC cell suspensions, 1x10⁶ cells/ml, in 96 well tissue culture plates. This mixture of cells and toxin was then incubated at 37°C under atmospheric conditions at 6% CO₂ for 72 hours. [³H]-thymidine (New Research Products, Boston, MA) at a concentration of 1 µCi/25 µl in a complete RPMI medium was added to each well and allowed to incubate under the same conditions for an additional 18-24 hours. After incubation, radiolabeled cells were harvested onto glass fiber filters (Skatron, Sterling, VA) using a semi-automatic cell harvester (Skatron). Lymphocyte proliferation was quantitated by measuring incorporation of [³H]-thymidine into cellular DNA using a liquid scintillation counter (TI-CARB 1500 Liquid Scintillation Counter, Packard, Rockville, MD).

Using SEC 1 wild type toxin as a control it was determined that the SEC1-12AA loop mutant toxin showed mitogenic activity at a reduced effectiveness compared to native SEC1 and the SEC1-4 and SEC1-9 mutants in stimulating PMBC cells (see Table 8).

**Table 8. SEC1 Loop Mutant Mitogeneicity [³H]-Thymidine Uptake (in CPM) of Enterotoxin Stimulated Human Peripheral Blood Mononuclear Cels**

| **Dose** | **SEC1** | **SEC1-4AA** | **SEC1-9AA** | **SEC1-12AA** |
|---|---|---|---|---|
| 0.1 pg | 1918 | 2067 | 1067 | 978 |
| 1 pg | 8533 | 7453 | 2360 | 954 |
| 0.01 ng | 14138 | 15495 | 7682 | 1200 |
| 0.1 ng | 21557 | 23689 | 21481 | 6500 |
| 1 ng | 30329 | 28892 | 28951 | 9400 |
| 0.01 µg | 33089 | 31640 | 23333 | 2700 |

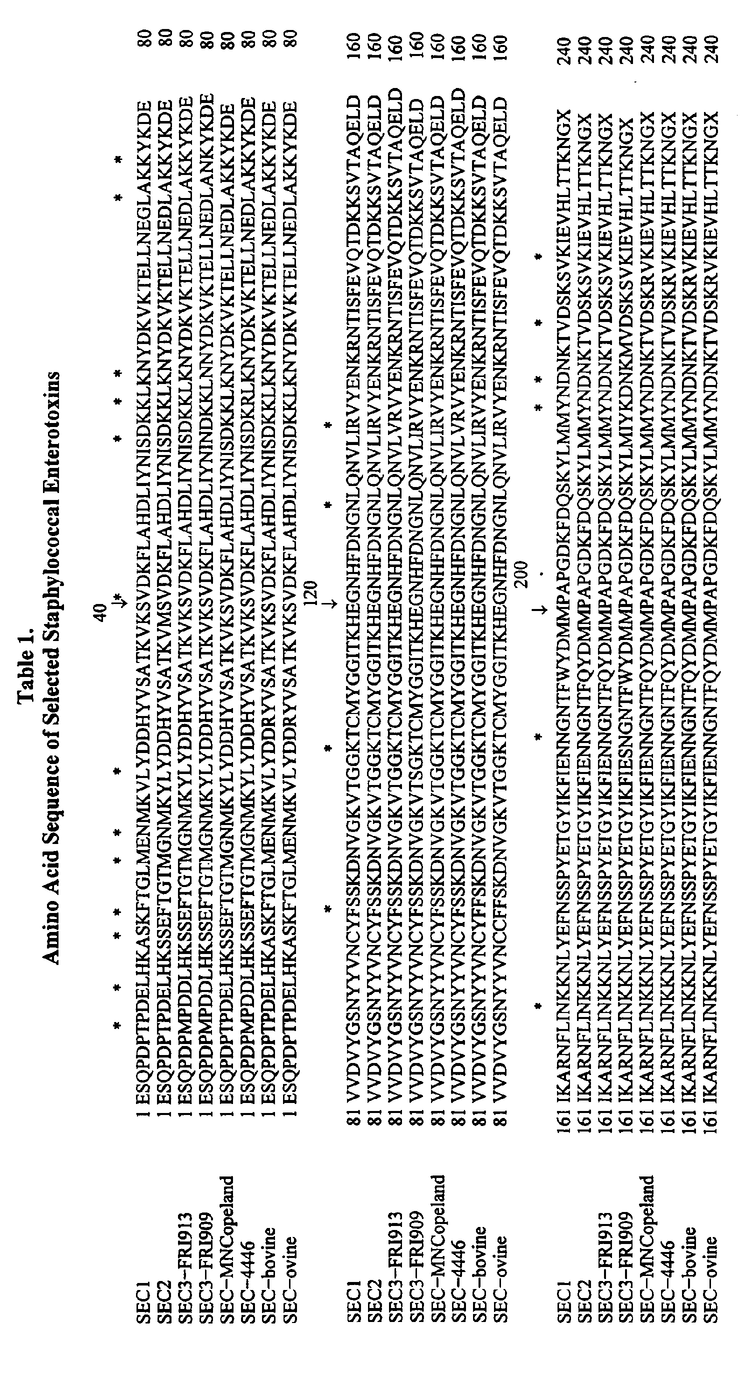

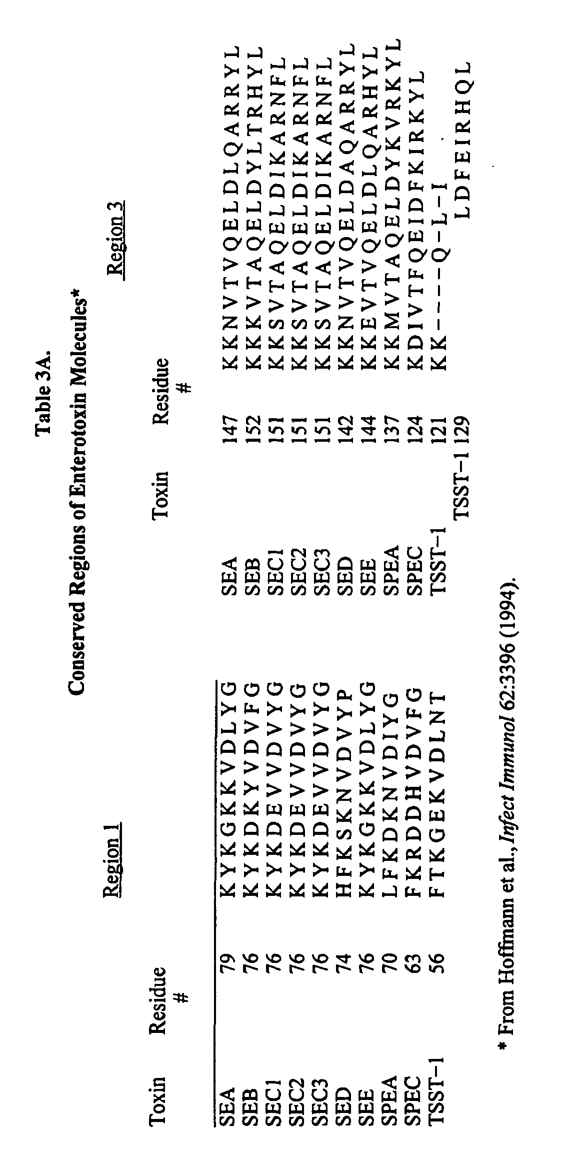

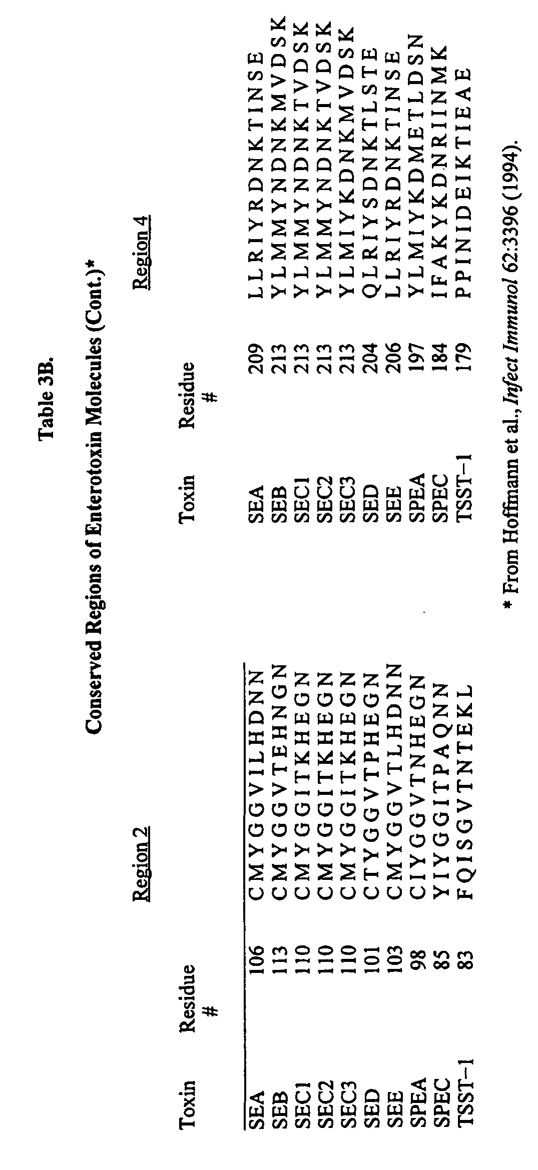

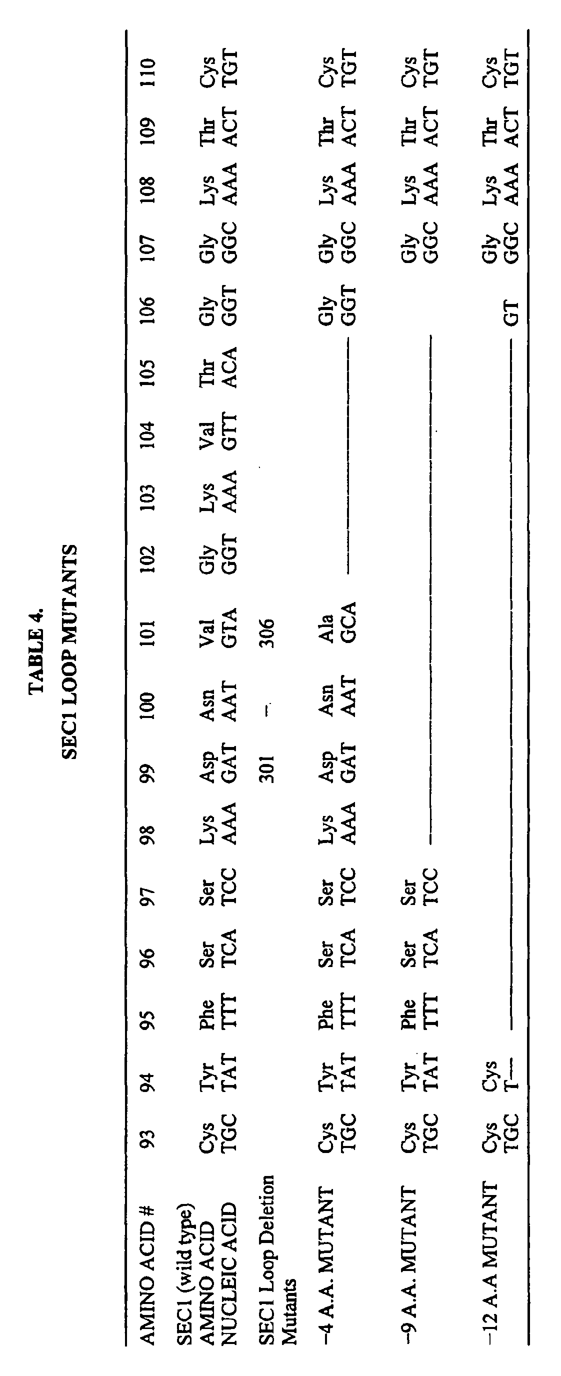

## Claims

1. A pyrogenic toxin derived from a native disulfide loop-containing pyrogenic toxin, wherein the toxin derived from a native disulfide loop-containing pyrogenic toxin is called modified toxin and comprises a disulfide loop region containing no more than 10 amino acid residues.

2. The modified toxin of claim 1 wherein the native disulfide loop-containing pyrogenic toxin is a staphylococcal toxin or a streptococcal toxin.

3. The modified toxin of claim 2 wherein the staphylococcal toxin is a type A, B, C, D, E, G or H staphylococcal enterotoxin.

4. The modified toxin of claim 2 wherein the streptococcal toxin is streptococcal pyrogenic exotoxin A or streptococcal superantigen.

5. The modified toxin of claim 1 wherein the disulfide loop region contains no more than 6 amino acid residues.

6. The modified toxin of claim 1 wherein the native disulfide loop-containing pyrogenic toxin is a type C staphylococcal enterotoxin.

7. The modified toxin of claim 6 wherein the modification comprises a deletion of at least 8 amino acid residues within the disulfide loop region.

8. The modified toxin of claim 7 wherein the modification comprises a deletion of amino acid residues 98-106.

9. The modified toxin of claim 6 wherein the modification comprises deletion of at least 12 amino acid residues within the disulfide loop region.

10. The modified toxin of claim 9 wherein the modification comprises deletion of amino acid residues 95-106.

11. The modified toxin of claim 6 wherein the type C staphylococcal enterotoxin is staphylococcal enterotoxin C1.

12. The modified toxin of claim 11 comprising a cysteine residue at position 94.

13. The modified toxin of claim 6 wherein the staphylococcal enterotoxin is staphylococcal enterotoxin C1, staphylococcal enterotoxin C2, staphylococcal enterotoxin C-MNCopeland, staphylococcal enterotoxin C-4446, staphylococcal enterotoxin C-bovine, staphylococcal enterotoxin C-canine or staphylococcal enterotoxin C-ovine.

14. The modified toxin of claim 1 comprising a cysteine residue at position 2 of the disulfide loop region.

15. The modified toxin of claim 1 having an emetic response inducing activity decreased by at least 100-fold in comparison to the native toxin.

16. The modified toxin of claim 1 having a fever inducing activity decreased by at least 100-fold in comparison to the native toxin.

17. The modified toxin of claim 1 having an LD₅₀ in Dutch Belted rabbits which is at least 100-fold higher than the native toxin.

18. A pyrogenic toxin derived from a native disulfide loop-containing pyrogenic toxin, wherein the toxin derived from a native disulfide loop-containing pyrogenic toxin is called modified toxin and comprises a disulfide loop region containing a deletion of at least 40 % of the amino acid residues within the disulfide loop of the native toxin.

19. The modified pyrogenic toxin of claim 18 comprising a cysteine residue at position 2 of the disulfide loop region.

20. The modified pyrogenic toxin of claim 18 wherein the native pyrogenic toxin is a type C staphylococcal enterotoxin.

21. An isolated nucleic acid comprising a nucleotide sequence encoding a pyrogenic toxin derived from a native disulfide loop-containing pyrogenic toxin, wherein the toxin derived from a native disulfide loop-containing pyrogenic toxin is called modified toxin and comprises a disulfide loop region which includes no more than 10 amino acid residues.

22. An isolated nucleic acid comprising a nucleotide sequence encoding a staphylococcal enterotoxin derived from a native disulfide loop-containing staphylococcal enterotoxin, wherein the staphylococcal enterotoxin comprises a disulfide loop region containing deletion of at least 40 % of the amino acid residues within the disulfide loop region of the native enterotoxin.

23. A pyrogenic toxin derived from a native type C staphylococcal enterotoxin, wherein the toxin derived from a native type C staphylococcal enterotoxin is called modified toxin and comprises a disulfide loop region which includes no more than 10 amino acid residues.

24. The modified toxin of claim 23 wherein the disulfide loop region is Cys-Cys-Gly-Lys-Thr-Cys.

## Patentansprüche

1. Ein pyrogenes Toxin, welches von einem nativen, Disulfidschleifen enthaltenden pyrogenen Toxin abgeleitet ist, wobei das Toxin, welches von einem nativen, Disulfidschleifen enthaltenden pyrogenen Toxin abgeleitet ist, als modifiziertes Toxin bezeichnet wird, und einen Disulfidschleifenbereich umfasst, welcher nicht mehr als 10 Aminosäurereste enthält.

2. Das modifizierte Toxin gemäß Anspruch 1, wobei das native, Disulfidschleifen enthaltende pyrogene Toxin ein Staphylokokken-Toxin, oder ein Streptokokken-Toxin ist.

3. Das modifizierte Toxin gemäß Anspruch 2, wobei das Staphylokokken-Toxin ein Staphylokokken-Enterotoxin vom Typ A, B, C, D, E, G oder H ist.

4. Das modifizierte Toxin gemäß Anspruch 2, wobei das Streptokokken-Toxin pyrogenes Streptokokken-Exotoxin A oder Streptokokken-Superantigen ist.

5. Das modifizierte Toxin gemäß Anspruch 1, wobei der Disulfidschleifenbereich nicht mehr als 6 Aminosäurereste enthält.

6. Das modifizierte Toxin gemäß Anspruch 1, wobei das native, Disulfidschleifen enthaltende pyrogene Toxin ein Staphylokokken-Enterotoxin vom Typ C ist.

7. Das modifizierte Toxin gemäß Anspruch 6, wobei die Modifizierung eine Deletion von mindestens 8 Aminosäureresten innerhalb des Disulfidschleifenbereichs umfasst.

8. Das modifizierte Toxin gemäß Anspruch 7, wobei die Modifizierung eine Deletion der Aminosäurereste 98-106 umfasst.

9. Das modifizierte Toxin gemäß Anspruch 6, wobei die Modifizierung eine Deletion von mindestens 12 Aminosäureresten innerhalb des Disulfidschleifenbereichs umfasst.

10. Das modifizierte Toxin gemäß Anspruch 9, wobei die Modifizierung die Deletion der Aminosäurereste 95-106 umfasst.

11. Das modifizierte Toxin gemäß Anspruch 6, wobei das Staphylokokken Enterotoxin vom Typ C das Staphylokokken Enterotoxin C1 ist.

12. Das modifizierte Toxin gemäß Anspruch 11, einen Cysteinrest an Position 94 umfassend.

13. Das modifizierte Toxin gemäß Anspruch 6, wobei das Staphylokokken-Enterotoxin Staphylokokken-Enterotoxin C1, Staphylokokken-Enterotoxin C2, Staphylokokken-Enterotoxin C-MNCopeland, Staphylokokken-Enterotoxin C-4446, Rinder-Staphylokokken-Enterotoxin C, Hunde-Staphylokokken-Enterotoxin C oder Schafs-Staphylokokken-Enterotoxin C ist.

14. Das modifizierte Toxin gemäß Anspruch 1, einen Cysteinrest an Position 2 des Disulfidschleifenbereichs umfassend.

15. Das modifizierte Toxin gemäß Anspruch 1, welches im Vergleich zum nativen Toxin eine mindestens um den Faktor 100 verringerte Brechreiz induzierende Aktivität besitzt.

16. Das modifizierte Toxin gemäß Anspruch 1, welches im Vergleich zum nativen Toxin eine mindestens um den Faktor 100 verringerte Fieber induzierende Aktivität besitzt.

17. Das modifizierte Toxin gemäß Anspruch 1, welches in Dutch Belted Kaninchen einen LD₅₀-Wert besitzt, der mindestens 100-fach höher ist, als der des nativen Toxins.

18. Ein pyrogenes Toxin, welches von einem nativen, Disulfidschleifen enthaltenden pyrogenen Toxin abgeleitet ist, wobei das Toxin, welches von einem nativen, Disulfidschleifen enthaltenden pyrogenen Toxin abgeleitet ist, als modifiziertes Toxin bezeichnet wird, und einen Disulfidschleifenbereich umfasst, welcher eine Deletion von mindestens 40% der Aminosäurereste innerhalb der Disulfidschleife des nativen Toxins enthält.

19. Das modifizierte pyrogene Toxin gemäß Anspruch 18, einen Cysteinrest an Position 2 des Disulfidschleifenbereichs umfassend.

20. Das modifizierte pyrogene Toxin gemäß Anspruch 18, wobei das native pyrogene Toxin ein Staphylokokken-Enterotoxin vom Typ C ist.

21. Eine isolierte Nukleinsäure, welche eine Nukleotidsequenz umfasst, die für ein pyrogenes Toxin kodiert, welches von einem nativen, Disulfidschleifen enthaltenden pyrogenen Toxin abgeleitet ist, wobei das Toxin, welches von einem nativen, Disulfidschleifen enthaltenden pyrogenen Toxin abgeleitet ist, als modifiziertes Toxin bezeichnet wird, und einen Disulfidschleifenbereich umfasst, welcher nicht mehr als 10 Aminosäurereste enthält.

22. Eine isolierte Nukleinsäure, welche eine Nukleotidsequenz umfasst, die für ein Staphylokokken-Enterotoxin kodiert, welches von einem nativen, Disulfidschleifen enthaltenden Staphylokokken-Enterotoxin abgeleitet ist, wobei das Staphylokokken-Enterotoxin einen Disulfidschleifenbereich umfasst, welcher eine Deletion von mindestens 40% der Aminosäurereste innerhalb der Disulfidschleife des nativen Enterotoxins enthält.

23. Ein pyrogenes Toxin, welches von einem nativen Staphylokokken-Enterotoxin vom Typ C abgeleitet ist, wobei das Toxin, welches von einem nativen Staphylokokken-Enterotoxin vom Typ C abgeleitet ist, als modifiziertes Toxin bezeichnet wird, und einen Disulfidschleifenbereich umfasst, welcher nicht mehr als 10 Aminosäurereste enthält.

24. Das modifizierte Toxin gemäß Anspruch 23, wobei der Disulfidschleifenbereich Cys-Cys-Gly-Lys-Thr-Cys ist.

## Revendications

1. Toxine pyrogène dérivée d'une toxine pyrogène contenant une boucle disulfure native, dans laquelle la toxine dérivée d'une toxine pyrogène contenant une boucle disulfure native est appelée toxine modifiée et comprend une région de boucle disulfure contenant au plus 10 résidus d'acide aminé.

2. Toxine modifiée selon la revendication 1, dans laquelle la toxine pyrogène contenant une boucle disulfure native est une toxine staphylococcique ou une toxine streptococcique.

3. Toxine modifiée selon la revendication 2, dans laquelle la toxine staphylococcique est une entérotoxine staphylococcique de type A, B, C, D, E, G ou H.

4. Toxine modifiée selon la revendication 2, dans laquelle la toxine streptococcique est une exotoxine pyrogène streptococcique A ou un superantigène streptococcique.

5. Toxine modifiée selon la revendication 1, dans laquelle la région de boucle disulfure contient au plus 6 résidus d'acide aminé.

6. Toxine modifiée selon la revendication 1, dans laquelle la toxine pyrogène contenant une boucle disulfure native est une entérotoxine staphylococcique de type C.

7. Toxine modifiée selon la revendication 6 dans laquelle la modification comprend une délétion d'au moins 8 résidus d'acide aminé dans la zone de boucle disulfure.

8. Toxine modifiée selon la revendication 7 dans laquelle la modification comprend une délétion de résidus d'acide aminé 98-106.

9. Toxine modifiée selon la revendication 6, dans laquelle la modification comprend la délétion d'au moins 12 résidus d'acide aminé dans la zone de la boucle disulfure.

10. Toxine modifiée selon la revendication 9, dans laquelle la modification comprend la délétion des résidus d'acide aminé 95-106.

11. Toxine modifiée selon la revendication 6, dans laquelle l'entérotoxine staphylococcique de type C est une entérotoxine staphylococcique C1.

12. Toxine modifiée selon la revendication 11 comprenant un résidu de cystéine en position 94.

13. Toxine modifiée selon la revendication 6, dans laquelle l'entérotoxine staphylococcique est une entérotoxine staphylococcique C1, une entérotoxine staphylococcique C2, une entérotoxine staphylococcique C-MNCopeland, une entérotoxine staphylococcique C-4446, une entérotoxine staphylococcique C-bovine, une entérotoxine staphylococcique C-canine, ou une entérotoxine staphylococcique C-ovine.

14. Toxine modifiée selon la revendication 1 comprenant un résidu de cystéine en position 2 de la région de boucle disulfure.

15. Toxine modifiée selon la revendication 1 ayant une activité induisant une réponse émétique diminuée d'au moins 100 fois par rapport à la toxine native.

16. Toxine modifiée selon la revendication 1, ayant une activité induisant de la fièvre diminuée d'au moins 100 fois par rapport à la toxine native.

17. Toxine modifiée selon la revendication 1 ayant un LD₅₀ chez les lapins Dutch Belted qui est au moins 100 fois supérieure à la toxine native.

18. Toxine pyrogène dérivée d'une toxine pyrogène contenant une boucle disulfure native, dans laquelle la toxine dérivée d'une toxine pyrogène contenant une boucle disulfure native est appelée toxine modifiée et comprend une région de boucle disulfure contenant une délétion d'au moins 40% des résidus d'acide aminé dans la boucle disulfure de la toxine native.

19. Toxine pyrogène modifiée, selon la revendication 18 comprenant un résidu de cystéine en position 2 de la région de boucle disulfure.

20. Toxine pyrogène modifiée, selon la revendication 18 dans laquelle la toxine pyrogène native est une entérotoxine staphylococcique de type C.

21. Acide nucléique isolé comprenant une séquence de nucléotides codant une toxine pyrogène dérivée d'une toxine pyrogène contenant une boucle disulfure native, dans laquelle la toxine dérivée d'une toxine pyrogène contenant une boucle disulfure native est appelée toxine modifiée et comprend une région de boucle disulfure qui comprend au plus 10 résidus d'acide aminé.

22. Acide nucléique isolé comprenant une séquence de nucléotide codant une entérotoxine staphylococcique dérivée d'une entérotoxine staphylococcique contenant une boucle disulfure native, dans laquelle l'entérotoxine staphylococcique comprend une région de boucle disulfure contenant une délétion d'au moins 40% des résidus d'acide aminé dans la région de la boucle disulfure de l'entérotoxine native.

23. Toxine pyrogène dérivée d'une entérotoxine staphylococcique de type C native, dans laquelle la toxine dérivée d'une entérotoxine staphylococcique de type C native est appelée toxine modifiée et comprend une région de boucle disulfure qui comprend au plus 10 résidus d'acide aminé.

24. Toxine modifiée selon la revendication 23, dans laquelle la région de boucle disulfure est Cys-Cys-Gly-Lys-Thr-Cys.
